# DEMANDE DE BREVET EUROPEEN

(11) **EP 3 913 911 A1**
(43) Date de publication de la demande: **24.11.2021**
(21) Numéro de dépôt: 20020157.2
(22) Date de dépôt: 06.04.2020
(51) Int. Cl.: H04N 7/15, H04N 7/14, H04L 12/18

(54) **SYSTÈME DE VISIOPHONIE SIMPLE**

(30) Priorité: 02.04.2020 WO PCT/EP2020/025159
(71) Demandeur: EDAGORA SOCIÉTÉ ANONYME, 75015 Paris (FR)
(72) Inventeur: EDAGORA SOCIÉTÉ ANONYME, 75015 Paris (FR)

(57) **Abrégé**

La présente invention concerne un dispositif et procédé de visiophonie, et se caractérise par l'absence de logiciel à installer ou de compte à créer par aucun des deux participants, une instantanéité de la mise en relation, et la simplicité pour l'invitant et pour l'invité.

Le dispositif comporte une infrastructure (1) auquel vient se connecter un terminal de l'invitant (6) qui fournit un identifiant (2) de l'invité. L'identifiant peut notamment être le numéro de téléphone portable de l'invité. Un lien unique (3) est transmis à l'invité par tout moyen numérique standard (4) tel SMS, email, MMS, WebPush normalisé, ne requérant pas d'installation de logiciel sur le téléphone de l'invité.

Lorsque l'invité clique sur ce lien, le navigateur internet de l'invité se connecte à l'URL unique (3), active la caméra et/ou le microphone du téléphone et établit un flux visio (5) avec l'invitant.

Dans une réalisation pratique, l'infrastructure (1) à disposition de l'appelant est une interface web avec un champ pour la saisie de l'identifiant (2) : le numéro de téléphone de l'invité. Dans une seconde réalisation, l'infrastructure (1) pourrait être un Serveur Vocal Interactif. Dans une troisième réalisation, l'identifiant (2) pourrait être l'adresse email et le moyen numérique standard (4) d'envoyer le lien pourrait être un email.

L'invention est susceptible d'application notamment lorsqu'un opérateur reçoit un appel entrant, que la vue de l'environnement de l'appelant est utile, urgente, et qu'on ne peut présupposer des éventuelles applications disponibles sur le téléphone de l'appelant.

L'invention peut être utilisée seule ou en supplément d'une conversation téléphonique (0), peut être enrichie de solutions de paiement, de collecte de données, et de transmission sécurisée de documents, et de transcription ou traduction simultanée.

## Description

La présente invention concerne un dispositif pour établir une communication de visiophonie, conversation en temps réel entre deux intervenants par le biais du son et de l'image.
La présente invention concerne également un procédé de communication de visiophonie.

Nous appellerons invitant (9) la personne qui souhaite amener l'autre à se joindre à elle dans une communication de visiophonie, et invité (10) la personne qui est ainsi sollicitée.

Le moyen traditionnel pour établir une telle communication dépend généralement d'un logiciel spécifique. Au titre de ces applications, citons Skype, Whatsapp, Facetime, Zoom. C'est en général depuis l'application que l'invitant déclenche une invitation. Celle-ci se manifeste de deux façons : soit elle réveille l'application de l'invité, qui déclenche une sonnerie et affiche un appel entrant ; soit elle se présente sous forme d'une URL textuelle et est transmise par tout moyen à l'invité, notamment email. Lorsque cette URL est cliquée par l'invité, l'application se lance.
Ce qui est remarquable c'est que l'invité et l'invitant doivent disposer de la même application, et qu'il est donc requis soit au préalable que l'invitant s'enquière de la disponibilité de l'application chez l'invité, soit que la communication soit retardée d'autant de temps qu'il faut pour que l'invité installe d'abord l'application, à supposer que cela soit possible (il faut que l'application soit disponible pour l'équipement, que l'invité ait les droits d'installation suffisants).

Le dispositif de l'invention vise à une communication instantanée et sans connaissance au préalable des applications en place chez l'invité. Par instantanée, on entend sans requérir l'installation d'une application, et sans enregistrement de l'invité dans un système (création de compte).

### Principe extrêmement simple d'établissement de la communication.

L'invité dispose d'un téléphone ou d'un appareil portable (8) muni d'une caméra, capable de communication numérique, et muni d'un navigateur internet. Par soucis de simplicité mais sans limitation aucune, nous appellerons cet appareil le téléphone. Le dispositif comporte par ailleurs une infrastructure (1) composée d'un serveur web assurant l'accessibilité d'un site internet auquel vient se connecter un terminal de l'invitant (6) (PC, tablette ou éventuellement terminal mobile). Cette infrastructure (1) est à disposition de l'invitant qui fournit à l'infrastructure (1) un identifiant (2) de l'invité. L'identifiant peut notamment être le numéro de téléphone portable de l'invité. Lorsque cette infrastructure (1) est activée en utilisant l'identifiant, un lien unique (3) est préparé afin d'accueillir l'invité. Ce lien est transmis à l'invité partout moyen numérique standard (4) tel SMS, email, MMS, WebPush normalisé (www.w3.org/TR/push-api/ et https://tools.ietf.org/html/rfc8030 et leurs évolutions futures) ou par websocket (https://tools.ietf.org/html/rfc6455 et évolutions futures), procédés ne requérant pas d'installation de logiciel sur le téléphone de l'invité.
Le lien unique (3) est tel qu'il n'est pas séquentiel et qu'il comporte une signature électronique empêchant les tentatives d'accès non autorisés.

Lorsque l'invité clique sur ce lien, le navigateur internet de l'invité établit la connexion avec l'URL unique (3). Dans cet URL est encodée une référence directe ou indirecte à l'invitant. Une référence indirecte peut être une référence de salle de réunion virtuelle dans laquelle attend l'invitant. Le navigateur accède alors à la caméra et/ou au microphone du téléphone et établit un flux visio (5) avec l'invitant. Cette étape ne requiert de la part de l'invité soit aucune action autre que le clic, soit au-delà du clic une seule action d'autorisation d'accès aux capteurs audio et/ou vidéo.

Une caractéristique essentielle de l'invention est la simplicité d'activation et d'interaction pour l'invitant :
- L'invitant n'a pas besoin de s'identifier sur l'infrastructure (1) pour les besoins de la communication visio
- L'invitant n'a pas besoin d'installer un logiciel, un navigateur internet suffit
- L'invitant n'a qu'une action très simple à faire sur l'infrastructure (1) : un seul champ à saisir et à valider - ou un correspondant à cliquer si l'implémentation fournit déjà une liste de correspondants.
- Dans une implémentation mono-directionnelle, l'invitant n'a pas besoin de matériel (microphone/caméra/carte son) ni d'autoriser l'accès à de tels périphériques.
- L'invitant n'a rien d'autre à faire après avoir activé l'infrastructure (1) : il patiente jusqu'à ce que la vidéo, et optionnellement le son, provenant du téléphone de l'invité lui soit (soient) restituée (restitués).

Une caractéristique essentielle de l'invention est la simplicité d'activation et d'interaction pour l'invité :
- L'invité n'a pas à s'identifier
- L'invité n'a pas de logiciel à installer, un navigateur internet suffit
- L'invité n'a rien d'autre à faire que de cliquer sur le lien et d'éventuellement accepter le partage de ses capteurs, le cas échéant.

### Exemples de réalisations pratiques

Dans une réalisation pratique,
- l'infrastructure (1) à disposition de l'appelant est une interface web avec un champ pour la saisie de l'identifiant (2)
- L'identifiant (2) est le numéro de téléphone de l'invité
- Le moyen numérique standard (4) d'envoyer le lien est un SMS ou un Webpush
- Le protocole utilisé pour l'établissement des flux de visiophonie (5) est le WebRTC, pilotés par des échanges Websocket.

Dans une seconde réalisation, l'infrastructure (1) pourrait être un Serveur Vocal Interactif.
Dans une troisième réalisation, l'identifiant (2) pourrait être l'adresse email et le moyen numérique standard (4) d'envoyer le lien pourrait être un email.

L'invention est susceptible d'application notamment dans les circonstances suivantes.
- Lorsqu'un opérateur reçoit un appel entrant d'une personne, et que l'assistance de l'opérateur ou le service fourni(e) par l'opérateur est grandement facilité(e) par la vue de l'appelant ou de l'environnement de l'appelant,
- et que l'établissement de la visiophonie est urgente
- et que l'opérateur ne peut disposer de l'information des éventuelles applications disponibles sur le téléphone de l'appelant
Dans ces cas de figure, l'opérateur est l'invitant et l'appelant est l'invité.
Il s'agit typiquement d'un appel vers un centre de traitement d'appels d'urgence médicale, sécurité ou incendie (112), violences conjugales, centre anti-poison, etc. L'implémentation peut dans ce cas rester mono-directionnelle avec tous les avantages de simplicité afférents.
- L'invitant et l'invité ont un rendez-vous et l'invitant ne connaît qu'un simple identifiant de l'invité (par exemple son numéro de téléphone).
   Il s'agit typiquement d'une consultation d'expert, consultation médicale, personnel soignant, psychologie, conseil, coaching, soutien, écoute.
- L'invitant souhaite éviter un déplacement pour observer une situation
Il s'agit notamment des constats d'experts, dans le cadre des litiges d'assurance, experts automobiles, des expertises et commissaires priseurs, des constats d'huissier.

Dans tous ces cas, la consultation visiophonique peut être utilisée seule, ou en supplément d'une conversation téléphonique (0) parallèle et indépendante notamment si l'implémentation est mono-directionnelle, choix qui peut être justifié par un besoin de simplicité et d'absence de requis matériels sur le terminal de l'invitant (micro, son, haut-parleurs). L'invitant peut disposer d'un contrôle permettant d'activer sur l'appareil portable le mode « mains libres » ou le volume sonore de la conversation téléphonique et/ou de la communication visio, et possiblement d'ajuster le volume du micro de l'appareil portable.
L'infrastructure (1) peut optionnellement être connectée à un équipement (13) de téléphonie PSTN/RTC (Public Switched Telephone Network / Réseau Téléphonique Commuté) ou VOIP (Voix sur IP) afin d'initier avant ou pendant la communication visio deux appels téléphoniques vers l'invité et l'invitant (qui aura précédemment du fournir son numéro dans cette implémentation à l'infrastructure (1)) afin de les mettre rassembler ou d'appeler l'un et de le transférer à l'autre, ou de réceptionner un appel et de le transférer à l'autre, ou de réceptionner deux appels des invités et invitants pour les réunir en communication, et ainsi permettre à l'infrastructure (1) d'établir automatiquement la conversation téléphonique, objet du paragraphe précédent.

### Implémentation technique, rôle de l'infrastructure (1)

- Lorsque l'invitant se connecte au site web, l'infrastructure (1) retourne une page HTML simple au navigateur internet du terminal qui affiche un formulaire simple de saisie de l'identifiant unique. L'invitant saisit l'identifiant et valide, ce qui retourne l'identifiant à l'infrastructure (1). Celle-ci alloue un numéro de communication visio, et renvoie un code Javascript assurant l'établissement d'une connexion websocket depuis le terminal vers l'infrastructure (1). Cette connexion permet à l'infrastructure d'initier une communication vers le navigateur internet du terminal (et non se contenter de son rôle de serveur).
- L'infrastructure (1) prépare une URL qui correspondra au numéro de communication visio ci-dessus, et l'envoie par le moyen standard. Par exemple si l'identifiant est une adresse email, le lien partira dans un email transmis à une passerelle SMTP, et si l'identifiant est un numéro de téléphone le lien sera dans le corps d'un message SMS remis à un fournisseur par le biais d'une API ad-hoc.
- Lorsque l'invité clique sur le lien, le navigateur internet de l'appareil portable se connecte au serveur web de l'infrastructure (1) qui lui retourne de façon similaire à l'invitant un code JavaScript assurant l'établissement d'une connexion websocket entre l'appareil portable et l'infrastructure (1).

- Ce même code JavaScript enchaîne avec la demande d'accès à la (les) caméra(s). En cas de succès, il active l'API de connexion Peer-to-Peer du navigateur internet qui génère une offre de connexion, un paquet SDP, qui est renvoyé via le websocket à l'infrastructure (1) qui le relaie au terminal.
- Le terminal fournit ce paquet SDP à l'API de Peer-to-Peer du navigateur internet. Ce paquet SDP contient les caractéristiques techniques des flux, des résolutions et encodages disponibles sur l'appareil portable. L'API compare les capacités de l'appareil portable et ses propres capacités, et constitue ainsi des propositions de paramétrages. La réponse SDP est relayées via Websocket à l'infrastructure (1) qui transmet à l'appareil portable.
- L'appareil portable choisit l'une des propositions de paramétrages et se met en attente de connexion des flux selon ce paramétrage, et renvoie son choix au terminal (toujours via websocket).
- Le terminal initie les flux. La connexion s'établit.
- Une fois la connexion peer to peer établie, elle sert pour acheminer les flux provenant des capteurs.
- Durant la communication visio, la connexion websocket reste active. Elle permet notamment de relayer les actions d'un côté qui affectent l'autre côté (contrôle des caméras).
- Lorsque l'une des partie termine l'appel ou ferme sa fenêtre de navigateur, l'autre partie détecte la perte du flux. Le Javascript est notifié et passe à l'étape suivante qui est l'affichage d'une page web avec un message adéquat par exemple.
La plupart des équipements sont placés derrière des pare-feux. Ceux-ci laissent passer les établissements de connexion TCP vers ure destination IP (adresse et port) dans un sens uniquement (sortant), sauf exceptions autorisées dans le sens entrant. Si les deux parties sont protégées par un pare-feux, et se retrouvent donc chacun à l'extérieur du pare-feux de l'autre, ni l'un ni l'autre ne peut établir de connexion TCP. Ce problème est résolu par l'intervention d'un serveur STUN (12) inclut dans l'infrastructure (1). L'adresse du serveur STUN est incluse dans le Javascript des deux côtés. Chaque partie s'adresse à ce serveur pour négocier des paramétrages convenables contournant les pare-feux pour une connexion en direct. Ces paramètres sont inclus dans les propositions et réponses SDP.

### Fonctionnalités connexes à la communication visio

Dans le cas où le téléphone de l'invité dispose de plusieurs caméras, l'invention permet éventuellement d'activer l'une, l'autre, ou les deux caméras simultanément, et de modifier cette configuration à souhait pendant la communication visio. L'implémentation choisie peut déléguer ce contrôle à l'invité et/ou à l'invitant, au moyen notamment de pictogrammes cliquables.
L'invention prévoit aussi de proposer un contrôle d'allumage ou d'extinction de la LED qui sert de lampe pour illuminer la scène obscure. Ce contrôle peut être fourni à l'invité mais aussi à l'invitant, ce qui permet à l'invité de ne pas dépendre de l'invitant pour ce paramétrage qui peut s'avérer complexe pour certaines personnes (notamment déstabilisées en situation d'urgence).

L'invention permet éventuellement à l'invitant et/ou à l'invité de saisir des photos pendant la communication visio, au moyens notamment de pictogrammes cliquables ou automatiquement en fonction d'un critère relatif au flux visio ou sonore, notamment : fort changement de luminosité, de contraste, changement de niveau sonore, dépassement d'un niveau sonore, présence ou absence d'un signal sonore. La prise de photos automatique peut également dépendre d'un signal généré par un accéléromètre embarqué dans le téléphone.
L'invention prévoit aussi de proposer un contrôle de l'activation du flash lors les prises de photo, paramétrage à la main de l'invité, mais aussi et surtout de l'invitant afin de préserver la simplicité côté invité.

L'invention permet éventuellement à l'invitant et/ou à l'invité d'enregistrer le ou les flux vidéo, en tout, en partie ou en multiples parties, au moyen notamment de pictogrammes cliquables ou de critères analogues à ceux du paragraphe précédent.

L'invention permet éventuellement de mettre ces enregistrements à disposition de l'invité et ou de l'invitant, selon choix d'implémentation, et éventuellement d'apposer une signature digitale cryptographique scellant l'observation avec éventuellement horodatage. Le résultat peut être disponible localement ou déposé sur un stockage en ligne.

L'invention prévoit aussi de donner le contrôle de la commutation des périphériques de sortie audio (haut-parleur d'oreille ou haut-parleur mains-libres) et éventuellement le réglage de volume de l'appareil portable.

### Autorisation de Webpush

Conformément aux dispositions du Webpush normalisé, l'accord de l'invité est indispensable pour enregistrer la possibilité d'adresser l'appareil portable. L'invention prévoit que le Javascript des deux côtés recueillir l'autorisation, avant ou après la communication visio selon l'implémentation. Cette autorisation est demandée au navigateur internet, qui présente la question à l'utilisateur selon une forme qui lui est propre. Dans le cas où cette autorisation est accordée, l'infrastructure (1) mémorise la correspondance entre l'identifiant (2) et le moyen ou identifiant d'adressage Webpush. À partir de ce moment, lorsque l'infrastructure (1) sera amenée à faire des choix sur le moyen de transport standard à employer, l'infrastructure pourra essayer le Webpush, et nous convenons d'appeler cela une situation où « le Webpush est disponible », étant précisé que la fonctionnalité de Webpush pouvant être indisponible aussi pour toute autre raison non liée à mémorisation de cette correspondance d'identifiants par l'infrastructure (1), notamment pour des raisons techniques tel qu'un rejet d'un serveur tiers à qui la demande de Webpush a été adressée, injoignabilité de ce serveur de Webpush, etc. auquel cas nous parlons d'« échec du Webpush ».

Ceci permet d'envoyer à l'appareil portable les messages suivants par Webpush quand disponible pour se substituer à un SMS qui engendre un coût :
- Toute autre invitation initiale à la visio communication telle que décrite depuis le début du document, émanant éventuellement d'invitants totalement disjoints (autre service, autre application, autre pays)
- Les informations de mise à disposition des documents post-communication visio (descriptions plus bas), leur mot de passe de déchiffrement, leur URL d'accès, ou bien même la data numérique les constituants si leur composition est implémentée dans le navigateur de l'invité.

De manière générale, toute évocation d'un envoi de SMS ou d'une autre notification standard (email) depuis l'infrastructure (1) et l'appareil portable peut être remplacée par un Webpush, si autorisé préalablement.

Il en résulte une économie de coûts intéressante notamment dans le cas de plusieurs usages disjoints de l'invention impliquant la même infrastructure (1). Néanmoins, la diversité des implémentations du Webpush dans les appareils portables ne permet pas systématiquement de s'assurer que l'infrastructure (1) est informée de la correcte délivrance du message Webpush. Pour palier à cette problématique, un mécanisme de timeout est prévu. Dans l'hypothèse où l'infrastructure (1) dispose de quoi communiquer par Webpush, le message (tel le lien unique (3) pour initier une visio) est envoyé par Webpush et un timer est déclenché. Si l'action attendue, telle le clic par l'invité sur le lien unique (3), est obtenue avant l'expiration du timer, le timer est arrêté et détruit. Si le timer expire, le message est à nouveau transmis par SMS.

La valeur du temps d'attente avant repli sur l'envoi pas SMS est un paramètre ajusté en fonction de l'urgence ou de l'importance de la communication visio. Pour une implémentation en centre de traitement d'appels d'urgences (médicales, incendie), le timer peut être rabaissé à zéro ou proche de zéro, engendrant un usage plus fréquent du SMS. Dans des cas moins sensibles, la durée d'attente peut être étendue pour maximiser les chances d'éviter un SMS.

À la fois les websockets et les Webpush permettent à l'infrastructure (1) d'adresser des messages aux deux navigateurs internets, mais les websockets ne restent connectés tant que la fenêtre du navigateur internet n'a pas été fermée. Les Webpush peuvent servir plusieurs semaines plus tard, mais la quantité d'information véhiculée est restreinte.

### Fonctionnalités de paiement

L'invention permet éventuellement à l'invitant ou à l'invité de requérir de la part de l'autre participant un paiement.

Ce paiement peut être demandé avant l'établissement des flux vidéo, c'est à dire lorsque le navigateur internet de l'invité traite le lien unique (3), tel un interstitiel (6) avant le déclenchement des flux. Cet interstitiel permettrait
- soit à l'invité d'établir une condition de paiement, notamment la saisie du montant demandé, amenant l'infrastructure de l'invitant (1) à proposer un parcours de paiement par le biais d'un moyen de paiement déjà renseigné ou d'un compte de pré-paiement préalablement alimenté, ou d'un moyen de paiement numérique selon éventuellement plusieurs choix proposés, et selon les pratiques de paiement en ligne régionales
- soit à l'invité d'autoriser un débit d'un moyen de paiement déjà renseigné ou d'un compte de pré-paiement préalablement alimenté, pour un montant indiqué par l'invitant sur l'infrastructure (1)
- soit à l'invité de procéder à un moyen de paiement numérique selon éventuellement plusieurs choix proposés, et selon les pratiques de paiement en ligne régionales, pour un montant indiqué par l'invitant sur l'infrastructure (1)

Il est entendu que cet interstitiel avant l'établissement des flux est aux dépens de la simplicité, cœur de l'invention, et est donc une implémentation subalterne. Le montant peut être une estimation maximale du prix qui sera défini à l'issue de la communication, auquel cas l'étape pré-visio est une autorisation de débit, et l'étape post-visio est l'utilisation de cette autorisation pour réaliser le paiement effectif.

Le paiement peut aussi être demandé à l'issue de la communication visio,
- soit dans la même fenêtre du navigateur internet de l'invité,
- soit par l'envoi d'un nouveau lien unique (3) par un moyen numérique standard (4), le même moyen ou éventuellement un moyen différent pour cet usage (il peut s'agir par exemple d'un email, si le premier lien unique (3) déclenchant la visio fut envoyé par SMS).
- soit par ré-utilisation du même lien unique (3). Dans ce dernier cas, l'invité peut simplement cliquer à nouveau sur le lien qu'il a reçu avant la communication visio, ou rafraîchir sa page. Le même lien activé avant la communication visio amène à établir la communication visio, mais activé après la communication il amène aux propositions de paiement et à un éventuel récapitulatif de la communication.
Le paiement à l'issue de la communication visio maintient la simplicité d'établissement des flux, propriété clé de l'invention.

Le paiement peut être demandé pendant la communication visio, auquel cas l'interface de paiement est présentée sur l'appareil portable sans interrompre la communication visio. Ceci permet un premier échange oral entre les intervenants qui permet au requérant de présenter son tarif, la prestation pouvant reprendre après paiement.

L'invention prévoit que l'une ou l'autre partie puisse se voir offrir la possibilité de demander un paiement à l'autre partie. Ainsi, bien que les exemples suivants mettent le paiement à charge de l'invité, dans d'autres configurations où l'invitant est le client d'un service fourni par l'invité l'invité précise sa demande de paiement selon l'une des options ci-dessus (avant, après ou pendant).

Le paiement peut prévoir différentes constituantes de prix qui peuvent être réparties au profit de différents intervenants, notamment l'invité ou l'invitant, l'opérateur de l'infrastructure (1), et l'éventuel interprète (voir plus bas).

### Collecte de données invité ou invitant

Dans la même implémentation, il est possible que l'invitant ait besoin de recueillir des informations de l'invité, ou réciproquement, qui dépendent du cas d'usage de l'implémentation. Par exemple, si la même implémentation est utilisée par des médecins, des experts automobiles, et des régulateurs de services d'urgence, l'invention permet à l'invitant de proposer des formulaires de collecte de données différents en fonction d'un paramétrage. Le formulaire proposé pour la collecte des données peut résulter d'une caractéristique de l'invitant (notamment sa profession), ou l'implémentation peut laisser l'invitant libre de définir les données à collecter, sous forme d'un formulaire libre.
- pour un médecin : nom prénom, numéro de sécurité sociale national, date de naissance, numéro de dossier d'assureur privé, nom du médecin traitant habituel
- pour un expert automobile : numéro d'immatriculation du véhicule...
- pour un service d'urgence : nom, prénom de l'invité et sa qualité de majeur, numéro de rappel
S'agissant de certains services où l'invité requiert des informations de l'invitant, il peut s'agir par exemple de services réservés aux majeurs : affirmation de sa qualité de majeur, date de naissance...

Cette collecte peut, de manière similaire à ce qui est précédemment exposé, apparaître en pré-communication ou post-communication, indépendamment du choix d'implémentation des demandes de paiement en pré-communication ou post-communication. Dans la situation où la demande de données et de paiement sont proposés conjointement (avant ou après la communication), l'implémentation peut choisir l'ordre ou éventuellement combiner l'ensemble dans une seule page.

### Chiffrage des données collectées

Afin d'alléger la responsabilité d'hébergement de données de l'invité ou de l'invitant, ou afin de garantir une protection des données de la vue d'autrui de bout en bout de ces données, qu'elles soient stockées sur l'infrastructure (1) de l'implémentation ou juste relayées, l'invention prévoit la génération en local d'une paire de clés asymétriques de part et d'autre (par usage des API de cryptographie des navigateurs internets), l'envoi à la partie opposée via le websocket de la clé dite « publique », l'usage par la partie opposée de cette clé pour crypter les données émises vers la partie initiale qui fera usage la clé qu'elle aura conservée secrète pour décrypter la donnée numérique avant la mise à disposition de l'invité ou mise à disposition de l'invitant (selon le sens).

L'invention prévoit l'envoi d'une clé dans chaque sens, mais l'implémentation peut porter sur un seul envoi en fonction de la confidentialité des données à recevoir en retour (ce qui sera le cas de l'exemple d'application décrit plus loin).

Si cet échange de clé est effectué suffisamment en amont, l'invention peut prévoir le stockage des éléments destinés à l'autre partie sous forme cryptés (et donc avec un risque quasi nul de compromission de données privées) sans avoir recours à des sécurisations sophistiquées de stockage, éventuellement même en ayant recours à des solutions de stockages dans le « cloud » grand public (Amazon Web Services, Google Drive...).

La possibilité de stockage permet le débrayage de la transmission de données, rendant les étapes de l'invité et de l'invitant décorrélées. Cela peut être particulièrement intéressant pour donner le temps à l'une des parties d'effectuer le paiement avant transmission des documents, lesquels documents peuvent déjà être préparés et disponibles sous leur forme chiffrée sur un stockage réseau.

Un exemple de réalisation pratique est l'usage de l'invention dans le cadre d'une consultation médicale ou avec un conseiller bancaire. Dans un cadre démuni de ce chiffrement, l'implémentation devrait obtenir l'agrément pour héberger les données transmises - même pour un stockage temporaire -, lesquelles données sont à remettre à l'autre partie après paiement de la consultation. Il n'est pas pratique de garder les deux parties en ligne pendant le temps du paiement, et le stockage des données à remettre contre paiement est nécessaire, faute de pouvoir imposer à son rédacteur d'attendre la réalisation du paiement.

### Intermédiaire de transcription

Dans certaines circonstances, notamment de handicap sensoriel de l'invité ou de l'invitant, l'implémentation peut proposer à l'invitant et/ou à l'invité l'intervention d'un transcripteur (11). Cette demande peut intervenir à l'initialisation de la communication visio, ou à tout moment en cas de besoin pendant cette communication. Dans ce cas, les flux prennent la forme de connexion bi-directionnelles en triangle, chaque partie recevant les flux des deux autres participants et le navigateur internet diffusant les flux depuis ses capteurs au deux autres participants.
L'interprète impliqué peut être soit un interprète maintenu disponible à la demande par le service de l'invitant, soit être choisi par l'invité sur le coup (interstitiel de choix) ou par un paramétrage préalable du client sur le service de transcription. Il peut être prévu une rémunération de l'interprète qui vient s'ajouter au prix proposé pour paiement (voir plus haut).
Par extension, il peut s'agir de n'importe quel conseiller externe ou traducteur, amenant effectivement à une visio consultation à trois.

### Consultation décalée dans le temps

L'invention prévoit la possibilité optionnelle de donner à l'invitant l'initiative de décaler dans le temps l'envoi du moyen numérique standard (4) contenant le lien d'invitation (3), notamment pour que celui-ci se déclenche à un moment prédéfini ou après l'écoulement d'une certaine durée. Cela peut être le cas quand l'invitant choisit l'invité dans une liste, tel en cliquant sur l'invité dans un annuaire en ligne et il est préférable de prendre RDV. Cela peut aussi être le cas d'une expertise automobile où il faut laisser du temps à l'invité pour qu'il se rendre proche du véhicule, objet du constat. Au moment d'établir la communication visio, l'infrastructure (1) détermine si un lien websocket est encore actif avec le terminal de l'invitant. À défaut de cela, un moyen numérique standard sera également utilisé au dit moment pour convier l'invitant à nouveau à la visio communication comme cela est fait pour l'invité : Webpush, Websocket, ou SMS. Naturellement, si il est envisageable que la connexion websocket ne soit plus active au moment d'établir la communication visio et que des cas d'usage du SMS sont amenés à se présenter, l'infrastructure (1) aura préalablement collecté le numéro de téléphone mobile de l'invitant, par exemple en même temps que celui-ci aura spécifié l'heure du rendez-vous ou le délai à attendre.

### Échange de documents confidentiels : préparation d'une clé ou paire de clés

À l'issue ou pendant la visio-communication, l'invention permet l'envoi de documents confidentiels à l'invité sans stockage en ligne en clair.
Dans une première implémentation, la paire de clés asymétrique évoquée ci-dessus servira au chiffrage et déchiffrage par le navigateur (par usage des API de cryptage du navigateur internet) avant et après la transmission, laquelle peut impliquer un stockage temporaire en ligne.

Dans une seconde implémentation, le chiffrage est assuré selon un algorithme de chiffrement réversible par une unique clé partagée. La solution assure le partage de cette clé de l'une des trois façons suivantes :
- L'infrastructure (1) génère une chaîne aléatoire avant, pendant, ou à l'issue de la communication visio et est transmise par websocket ou dans le Javascript initial, lequel aux deux extrémités en assure l'affichage pendant toute la durée de communication visio sur l'écran, ou sur l'écran qui succède au raccroché : « Notez bien votre clé de suivi pour les échanges de documents ultérieurs : [chaîne aléatoire]. Elle vous sera demandée pour déchiffrer les documents. »
- En supplément ou alternativement à la précédente, la chaîne aléatoire peut être envoyée à l'invité par SMS, ce qui lui en donne une trace rémanente sans avoir à noter. Elle peut même être incluse dans le SMS d'invitation (4) contenant le lien à cliquer avant la communication visio, ceci présentant l'avantage d'économiser un SMS ultérieur. « Cliquez sur le lien .... Par ailleurs, votre mot de passe pour l'échange ultérieur des documents est ... ».
- L'implémentation peut prévoir que la partie variable du lien unique 3 soit la clé du chiffrage réversible
La valeur de cette clé peut être stockée localement par le navigateur internet de l'appareil portable, ce qui permet de l'afficher à nouveau à l'invité. Pour ce faire, il suffit que l'invité accède à une URL de l'infrastructure (1) qui retourne un code Javascript qui sait retrouver la clé dans le stockage local et l'afficher. La clé n'a pas à quitter l'appareil portable.
Cette implémentation ne couvre pas la transmission des documents chiffrés eux-même, lesdits documents peuvent être transmis par email ou par service web de transfert de document, puisqu'ils ne sont pas utilisables sous leur forme chiffrée. Néanmoins l'invention prévoit de mettre à profit les différents éléments exposés pour proposer une solution.

### Échange de documents confidentiels : transmission des documents

Comme indiqué, les documents chiffrés sont aptes à être envoyés par email ou déposés sur un stockage en ligne. L'infrastructure (1) peut fournir ce service d'hébergement de la donnée chiffrée ou faire appel à un hébergement web tiers (Amazon Web Services, Google Drive). Dans ce cas, l'infrastructure (1) upload la donnée chiffrée, détermine l'URL de téléchargement du paquet chiffré, et la transmet à l'invité par l'un des moyens de transmission standard évoqués, notamment Webpush, emails ou SMS à défaut. Le navigateur internet chargera la donnée chiffrée et deux cas de figure se présentent.

Dans la seconde implémentation (celle à clé partagée), le document est typiquement exploitable tel quel, tel un PDF chiffré. L'outil d'ouverture du document (Acrobat Reader) se charge de l'ergonomie de saisie d'un mot de passe.
Dans la première implémentation (à clés asymétriques), ou si un chiffrement propriétaire est appliqué dans la seconde implémentation (par opposition au mécanisme prévu par la norme PDF), c'est un script local (application web, JavaScript...) qui peut se charger du déchiffrage à partir de la clé (privée ou partagée) à l'insu de l'utilisateur, et déposer le fichier déchiffré dans l'endroit de dépose habituel du navigateur.
Dans un cas particulier, les données variables du document sont les seules données transférées, séparées du reste du document tel un démembrement. On nommera « données démembrées » les données variables du document, et « document générique » le complémentaire, l'union des deux permettant de reconstituer le document final, égal au document de départ. Les données démembrées peuvent être les données prévues pour remplir les champs d'un formulaire PDF, ou du texte d'un courrier sans la lettre type constituée de son en-tête, son pied de page et son éventuel fond graphique. La lettre type ou le formulaire PDF sont les documents génériques et ils peuvent être mis en ligne sous une URL car ils sont démunis de données sensibles. Les données sensibles et l'URL du document générique sont chiffrées par l'un des deux moyens ci-dessus, et le paquet chiffré transmis à l'infrastructure (1) jusqu'ici comme plus haut. L'intérêt de cette méthode est d'alléger la partie de données au maximum. L'infrastructure (1) qui réceptionne les données démembrées cryptées peut alors utiliser le Webpush pour apporter les données au navigateur internet de l'appareil portable. Un second Webpush notifie l'utilisateur de la réception de ses documents. Lorsque l'invité clique sur cette notification, le navigateur internet, dispose de l'URL du document générique et des données démembrées chiffrées. Le code Javascript télécharge le document générique, déchiffre le paquet de données, combine les deux, et reconstitue (remembrement) le document, puis le dépose à l'endroit habituel des documents téléchargés.

### Échange de documents nécessitant une protection à la falsification

L'invention prévoit deux mécanismes pour protéger les documents contre une falsification : un document de contrôle, et l'adjonction de hash de vérification.

Le premier mécanisme requiert un stockage en ligne d'une version suffisamment banalisée du document pour qu'il perde sa caractéristique de confidentialité. Nous appelons cette version le « document de contrôle ». Il ne contient pas de données sensibles et peut donc après création par le Javascript du terminal, transmission à l'infrastructure, être hébergé en ligne par l'infrastructure (1).
L'original en clair, transmis à l'invité et typiquement imprimé par celui-ci, comporte une URL d'accès au document de contrôle, en format textuel et en codage optique tel que QR-code. Le contrôle consiste à saisir ou scanner l'URL et ainsi afficher le document de contrôle, et à comparer visuellement l'original au document de contrôle.
Pour constituer le document de contrôle, les mécanismes de banalisations exécutés par le Javascript du terminal qui poste ensuite son résultat à l'infrastructure (1), peuvent être par exemple et sans limitation :
- le remplacement d'un certain nombre de caractères ou de certains caractères par un symbole (à la manière dont les mots de passe informatiques sont occultés), par exemple tout caractère sauf les premiers de chaque mot et les chiffres.
- un floutage suffisant pour laisser apparaître la longueur des mots sans qu'ils ne soient lisibles.

Le second mécanisme ne requiert aucun stockage en ligne. Il s'agit d'effectuer une signature de contrôle - hash - sur les caractères de la page (ou d'autant de parties de pages que souhaité), de signer ces hash (par une clé privée) et de les faire apparaître sur le document. Le contrôle consiste à scanner le document, à utiliser une conversion par reconnaissance de caractères pour isoler les hash signés du reste du document, à vérifier l'authenticité des hash signés (par une clé publique), puis à recalculer les hash à partir du reste du texte du document et à les comparer aux versions apparaissant sur le document.

### Échange de documents à usage compté

Un identifiant unique, tel un UUID, peut être apposé sur les documents. Dès lors, un service en ligne, éventuellement accessible à partir d'une URL imprimée sur le document sous forme textuelle et codage optique (QR), permet de s'assurer que le document n'a été présenté qu'une fois ou un nombre de fois convenable à un point de contrôle. Le premier mécanisme de résistance à la falsification ci-dessus étant déjà une URL, celle-ci peut servir aux deux besoins simultanément.

### Exemple concret d'implémentation. version étendue

Cet exemple prend la situation d'une consultation médicale. Cet exemple n'est pas restrictif à ce domaine mais illustratif. La consultation médicale peut être remplacée par un conseil financier ou tout autre domaine impliquant aussi des données confidentielles et l'échange de documents sensibles à l'issue de la communication visio.

### Préalables

- Du fait de l'activité du médecin, un paramétrage de la solution indique que les données à collecter sont le nom, prénom, date de naissance et numéro de sécurité sociale du patient.
- Le médecin s'est enregistré auprès d'un organisme de traitement de paiements sur compte de cantonnement, organisme tiers dénommé PSP (7) disposant pour cela d'un agrément d'une autorité prudentielle.

L'infrastructure (1) est un site web auquel le médecin se connecte (en https). Dans le cas d'un service gratuit (urgentiste), l'identification et l'inscription auprès de l'organisme tiers agréé ne sont pas nécessaires.

### Infrastructure technique (1)

L'infrastructure technique repose sur
- un serveur web communiquant en protocole HTTP/HTTPS (par exemple Apache + Nginx) et disposant de certificats SSL émanants d'un CA notoire,
- un serveur établissant des connexions websocket entre un serveur (possiblement le même serveur que le serveur web) et les navigateurs web des invitants et invités,
- une signalétique transitant par websocket
- des flux conformes à la norme WebRTC
- un serveur STUN/TURN permettant l'établissement de ces flux au travers de pare-feux réseaux par l'échange de paquets SDP
Les navigateurs internets se connectent au serveur websocket au chargement des pages HTML requérant la visio communication.
Quand il s'agit d'initier un flux vidéo, les navigateurs internet exécutent du code JavaScript et envoient des données par websocket au serveur qui transmet ces données et permet aux navigateurs internet de s'accorder sur les paramètres de la connexion de flux au standard WebRTC.
Les connexions websockets restent actives tant qu'il y a des besoins de signalétique et au-delà pour les besoins de signalétiques à l'initiative du serveur web, tant que la fenêtre du navigateur internet reste ouverte sur l'application (URL du site).

### Identification

Dès lors qu'il est prévu un paiement, le médecin commence par s'identifier. Dans cet exemple, cela se fait au moyen de son adresse email. L'infrastructure sonde le PSP pour l'existence d'un compte de paiement. Si tel compte est trouvé, l'infrastructure (1) envoie à l'adresse email un lien contenant une signature. Le médecin clique sur ce lien, et revient sur le site avec la signature qui atteste qu'il est légitime détenteur du compte email et donc du compte de paiement chez le PSP. L'infrastructure (1) stocke dans un cookie du navigateur cette adresse email ou un identifiant de compte, et une signature horodatée permettant à l'infrastructure (1) de s'assurer ultérieurement que l'identification a été faite et qu'elle est récente selon un seuil acceptable.

### Consultation

Le médecin est possiblement déjà en consultation téléphonique (0) avec le patient, notamment s'il s'agit d'un médecin urgentiste. Il connaît, ou copie depuis son logiciel métier, ou demande au patient avec qui il discute au téléphone, le numéro de mobile du patient.
Le médecin dispose d'une interface web extrêmement simple, ne présentant qu'un champ de saisie dans cet exemple. Il colle ou tape le numéro de mobile du patient et valide. Aucune autre interaction de sa part n'est requise, il verra apparaître le flux visio.

L'infrastructure détermine un numéro de consultation, met le navigateur du médecin en attente de l'établissement de cette communication, prépare un lien unique signé rattaché de façon bijective à la consultation, et envoie ce lien par SMS au patient.
Si et seulement si le médecin est identifié et que le PSP est garant de l'activité légitime de médecin (inscription à l'ordre national des médecins), le texte du SMS inclut une annonce avec le nom du médecin (« Dr X vous invite à cliquer sur ce lien ») pour rassurer le patient. L'infrastructure prépare un mot de passe, l'ajoute à ce SMS (« Votre mot de passe pour les documents après la consultation est:... ») et affichera ce mot de passe au médecin pour s'il souhaite chiffrer ses documents pas ce moyen simple (outre le système de clés asymétriques décrit plus bas).

Le patient clique sur le SMS sans interrompre l'éventuel appel en cours. Cela lance le navigateur internet de son appareil portable qui lui demande éventuellement l'autorisation d'accéder à sa caméra et son microphone, ce que le patient accepte. À cette éventuelle autorisation près, le patient n'a absolument aucune autre action à mener. Lors d'un usage futur, le navigateur aura mémorisé son autorisation et la seule action requise sera le clic sur le lien reçu par SMS. La communication visiophonique est établie.

Le patient et le médecin disposent, dans cet exemple, de pictogrammes pour activer l'une, l'autre, ou les deux caméras du téléphone. Dans le cas de deux caméras actives, le médecin peut choisir d'avoir les deux flux côte à côte ou celui qui est affiché sur le téléphone en plus grand. Le médecin dispose d'un bouton pour photographier et enregistrer différentes séquences qui seront disponibles ensuite dans le répertoire dans lequel le navigateur dépose usuellement les documents téléchargés. D'autres implémentations et lieux de stockages seraient possibles, notamment en réseau, mais pas dans cette implémentation. Le médecin dispose d'un bouton pour allumer la lampe torche de l'appareil portable. Le médecin dispose d'un contrôle du passage ne haut-parleur mains-libres et éventuellement du volume de l'appareil portable.

L'appel téléphonique éventuellement préexistant peut rester actif pendant toute la consultation visio, à la demande du médecin.

Le patient et le médecin disposent d'un bouton pour faire intervenir un interprète en Langue des Signes ou un transcripteur écrit si le patient est sourd, auquel cas l'écran de l'appareil portable dédiera l'espace principal à l'interprète en Langue des Signes, ou au texte transcrit. L'interface du médecin présentera les deux intervenants côte à côte.

### Paiement

Lorsque l'un ou l'autre actionne le bouton de fin de communication, l'implémentation de cet exemple prévoit
- dans le cas d'un médecin non identifié, d'un urgentiste, ou d'une consultation gratuite, le retour simple à l'écran permettant d'initier la communication visio avec un autre patient. Le process reste extrêmement simple et s'arrête là.
- dans un cas d'un médecin identifié chez un PSP (selon cet exemple), l'implémentation de cet exemple prévoit que le médecin saisisse un prix de consultation, une codification de son acte, un texte d'ordonnance médicale ou de certificat médical, des données d'arrêt maladie. Il peut commencer à saisir ces éléments sans attendre la fin de la communication visio.
Sur validation par le médecin au moins du prix de paiement, l'infrastructure (1) détermine si le navigateur du patient est toujours disponible et connecté par websocket à l'infrastructure (1), auquel cas, ou par l'intermédiaire d'un WebPush normalisé (www.w3.org/TR/push-api/), le navigateur du patient charge une page contenant un formulaire
- requérant les données souhaitées et dépendant de la profession (voir section « préalables » : nom, prénom, date de naissance et numéro de sécurité sociale du patient)
- affiche le prix demandé par le médecin
- propose via un curseur de laisser un pourboire supplémentaire pour l'opérateur de l'infrastructure (1)
- un bouton d'envoi
Si le navigateur de patient n'est plus connecté par websocket à l'infrastructure (1) ou joignable par WebPush, l'infrastructure envoie un nouveau SMS au patient avec un lien (identique ou différent) et un nouveau message l'invitant à cliquer sur le lien, ce qui amènera au formulaire ci-dessus.
Pendant ce temps, le médecin peut continuer ou modifier les autres saisies (codification de son acte, texte d'ordonnance médicale ou de certificat médical, données d'arrêt maladie). Ces données sont stockées localement via l'API de stockage local du navigateur internet, dans cette implémentation.

### Traitement du paiement

Lorsque le patient valide son formulaire avec le prix et ses données
- le navigateur génère une paire de clés asymétriques, conserve celle dite privée dans son stockage local (API de stockage du navigateur internet), adjoint celle dite publique au formulaire
- transfère les données à l'infrastructure (1) qui prépare une URL de paiement chez le PSP, prenant soin de rattacher toutes les données reçues à ce paiement
- redirige le navigateur internet du patient vers les interfaces de paiement du PSP
- lors du traitement par le PSP, les différents constituants du montant du paiement sont répartis au profit des différents intervenants dans des comptes de cantonnement dédiés.
Sur paiement validé, le PSP notifie l'infrastructure (1) qui, si elle détecte une connexion ouverte en websocket avec le navigateur internet du médecin, notifie le navigateur internet du médecin de cela, résultant dans une information au médecin, ou utilise un mécanisme de webpush normalisé (www.w3.org/TR/push-api/) pour informer le médecin. Mais cette information reste optionnelle. Elle invite le médecin à aller sur une page d'historique de consultations.

### Historique de consultation

Cette page du site n'est disponible que si le médecin est authentifié (dans notre exemple, authentifié chez le PSP). Lorsque le médecin y accède, l'infrastructure (1) reçoit en cookie l'email ou l'identifiant de compte auprès du PSP, ainsi que sa signature d'intégrité qu'elle vérifie. Lorsque cette donnée est inaltérée et correspond aux valeurs que l'infrastructure avait posées lors de l'identification, alors la liste des transactions de paiement est récupérée auprès du PSP, grâce à l'adresse email ou à l'identifiant de compte.
Le navigateur internet affiche une liste de consultations, et leur statut de paiement, ainsi qu'un moyen d'afficher ou d'utiliser les données utilisateur saisies par le patient et rattachées à la demande de paiement.

### Exploitation des données client par le médecin

Ces données utilisateur peuvent être simplement affichées pour que le médecin puisse les copier coller dans d'autres outils. Alternativement et de manière plus élaborée, une librairie JavaScript permet de combiner les données client et les données locales saisies par le médecin pour remplir des formulaires normalisés PDF et présenter (ou pas) au médecin un ou plusieurs PDF final/finaux.
Le médecin peut utiliser les mécanismes de signature de PDF grâce à un certificat numérique commercial dont la chaîne de validation certifie son identité. Il peut aussi utiliser le mot de passe que l'infrastructure (1) lui a affiché pour verrouiller l'ouverture du PDF. Dans ce cas simple, il exploite uniquement des mécanismes de protection fournis par les éditeurs de PDF.

### Fourniture des documents au patient

Sur validation du médecin, le ou les PDF sont cryptés à l'aide de la clé publique générée sur le navigateur internet du patient. Elles sont alors transmises à l'infrastructure (1) qui en assure le stockage éventuellement par l'usage de stockages tiers de type Amazon Web Services.
Si la connexion websocket avec le navigateur du patient est encore ouverte, ou si l'usage de Webpush normalisé est possible, l'infrastructure (1) notifie le patient de la mise à disposition de ses documents. À défaut, un lien est envoyé (même URL ou URL différente) par SMS ou par email si l'adresse est disponible (notamment si elle est recueillie par le PSP), amenant dans tous les cas le navigateur internet du patient à accéder à la donnée cryptée, à la déchiffrer à l'aide de sa clé privée récupérée dans le stockage local du navigateur, et à mettre à disposition tel un téléchargement standard le ou les documents dans le répertoire habituel de stockage des pièces téléchargées. Comme évoqué plus haut, une autre implémentation ne transférera (idéalement par Webpush ou à défaut par mise en ligne sous une URL à laquelle l'appareil portable accède) que les données sensibles et une référence de document vierge (template, formulaire PDF, fond de page) et la reconstitution s'effectuera par le navigateur internet de l'invité.

Dans cette implémentation, l'infrastructure (1) ne stocke aucune donnée médicale. Si les données patient (le nom, prénom, date de naissance et numéro de sécurité sociale du patient) étaient amenées à être considérées comme données médicales, l'implémentation inclurait une génération de paire de clés dans le navigateur internet du médecin, l'une des clés accompagnant la demande de paiement pour servir au chiffrage des données saisies par le patient. Il nous a semblé judicieux dans cet exemple de ne faire apparaître le chiffrage que dans un sens, pour en montrer le côté optionnel.

Dans cette implémentation, l'infrastructure (1) ne stocke même pas de données de compte de médecin (login, mot de passe), délégant au PSP la validation d'un professionnel de santé. Ceci est un choix dans cet exemple pour montrer qu'il est possible de fonctionner sans le moindre stockage de données dans l'infrastructure (1) ni même compte client pour l'invitant. La liste des consultations et les données associées sont hébergées par le PSP, comme métadonnées d'un paiement. Ces données dans notre exemple ne sont pas de nature médicales, mais si elles étaient considérées sensibles elles seraient toujours stockées chez le PSP sous une forme chiffrée. Les données médicales sensibles (ordonnance, etc...) ne sont stockées que localement par le navigateur internet (via son API de stockage local) du médecin.

### Usage de documents imprimés - Ordonnances

Dans la poursuite de cet exemple, le patient imprime son ordonnance et l'apporte à la pharmacie. Faute d'avoir un document numérique (signature numérique du médecin et possibilité de vérifier l'authenticité de la prescription, la correspondance à l'original), il est important de fournir au pharmacien d'une part un moyen de s'assurer que l'ordonnance n'a pas été modifiée, et d'autre part qu'elle n'a pas déjà été traitée par un autre pharmacien.
Les principes actuels reposent sur une vérification de la signature du médecin sur un document original, puis l'apposition d'un tampon du pharmacien sur l'original qui ne peut donc plus donner lieu à délivrance des médicaments dans une autre pharmacie.

Pour palier à cela, l'invention prévoit deux mécanismes séparés mais cumulables : un document de contrôle ou des hash de vérification. Ces mécanismes sont décrits ici pour les ordonnances, mais sont généralisables aux autres documents de l'exemple (arrêt de travail, certificat médical, feuille de soins, déclaration d'accident du travail...), ou à tout autre document dans d'autres usages de l'invention.

### Document de contrôle

En outre des documents remontés par le navigateur internet du médecin à l'infrastructure (1) (PDF signés et chiffrés par le médecin, ou par l'application s'exécutant dans le navigateur internet du terminal, ou juste du paquet de données chiffrées si le remembrement a lieu sur l'appareil portable) , le navigateur internet du terminal prépare une copie de l'ordonnance dans un document PDF (ou image) de contrôle dans lequel tous les caractères de A à Z, majuscules comme minuscules, sont remplacés par un caractère opaque (un cercle plein par exemple à l'instar de l'obfuscation des saisies de mots de passes) à l'exception du premier caractère de chaque mot. Les chiffres ne sont pas remplacés. De façon optionnelle les majuscules sont toutes remplacées par des minuscules.
Ce document est transmis à l'infrastructure (1) non chiffré car il ne contient plus de donnée médicale, les premières lettres n'étant pas suffisantes pour identifier les médicaments dans une phrase dont on ne peut supposer certain qu'elle commence par le nom du médicament. Le numéro de sécurité sociale et les initiales du patient sont en clair. Le nombre de boîtes ou de comprimés sont en clair, sans qu'on ne puisse savoir s'il s'agit de boîtes, de comprimés, ou de fréquence de prise. Le document ne dévoile aucune donnée médicale effective, mais néanmoins suffisamment de données qu'un utilisateur malintentionné souhaiterait modifier. Ce document peut donc être rendu accessible sur internet par une URL (éventuellement comprenant une signature), document hébergé par l'infrastructure (1) ou dans un service de stockage tiers.
L'URL est telle qu'une brute force ne permet pas d'obtenir les documents par des personnes non autorisées.
Cette URL est inscrite sur l'ordonnance médicale (l'ordonnance chiffrée et transmise au patient, imprimée, apportée en pharmacie), en textuel ainsi qu'en QR-code ou tout autre codage optique. Le pharmacien peut ainsi afficher dans son navigateur internet le document de contrôle, et vérifier visuellement que l'ordonnance n'a pas été altérée en vue de changer les posologies ou de modifier les médicaments prévus.
Sur accès à cette version en ligne du document de contrôle, le pharmacien peut alors passer de façon irréversible l'ordonnance dans un statut livrée. Ceci permet à tout autre pharmacien d'être alerté si le patient utilise une autre impression de son ordonnance.
La signature de l'URL mentionnée ci-dessus n'est pas indispensable. Elle permet juste d'éviter que le système soit interrogé de façon malveillante avec des paramètres aléatoires. De préférence, une signature à clé asymétrique serait utilisée, la clé privée ne résidant qu'au cœur du service répondant à l'URL d'accès.

### Hash de vérification

Le navigateur du patient calcule une signature (hash) du document et l'inscrit sur le document, encadré par une signalétique permettant de l'isoler dans la reconnaissance optique.
Sur présentation de l'ordonnance à un pharmacien, celui-ci la scanne et utilise un mécanisme de reconnaissance de caractères (OCR) qui alimente le même algorithme de hash (exclusion faite du hash lui-même dont un marqueur graphique indique l'emplacement). La clé doit correspondre, ou alors le pharmacien passe à des vérifications supplémentaires manuelles (appel du médecin, de la même manière qu'en cas de doutes sur l'authenticité d'une ordonnance manuscrite).
Un raffinement de ce mécanisme consiste à mettre plusieurs hash, par exemple un par quadrant, afin d'identifier les parties intactes et celles dont la vérification échoue. Un second passage peut s'avérer plus fructueux.
Ce mécanisme ne requiert aucun stockage de donnée médicale en réseau. L'outil d'OCR et de recalcul du hash peut être soit local, soit distant sur un service web.

### Identifiant unique référencé sur un service centralisé

Pour palier à l'autre problème, l'usage multiple de cette ordonnance imprimée plusieurs fois auquel le hash de vérification ne pallie pas, le document peut faire l'objet d'une attribution d'un numéro unique (ou à très haute probabilité d'être unique) de type UUID (RFC 4122). Cet identifiant est encapsulé dans une URL qui est inscrite en clair (adjointe d'une éventuelle signature) et en QR-code ou tout autre codage optique sur le document. Lors du premier accès à cette URL, le statut de ce document passe de façon irréversible à un état « traité » ou incrémente un compteur et sauvegarde éventuellement d'autres données (adresse IP, identifiant du pharmacien).
Tout accès ultérieur à cette URL engendre un affichage d'alerte, ou l'affichage du nombre de flashages et d'accès de cette URL ou toute autre donnée pertinente (identifiant du pharmacien ayant précédemment traité l'ordonnance).
La signature de l'URL mentionnée ci-dessus n'est pas indispensable. Elle permet juste d'éviter que le système soit alimenté de façon malveillante par un grand nombre aléatoire d'identifiants. De préférence, une signature à clé asymétrique serait utilisée, la clé privée ne résidant qu'au cœur du service centralisé.

Les protections précédentes sont cumulables. Elles sont généralisables à tout document dont le stockage en ligne n'est pas souhaité mais dont l'intégrité doit être vérifiable dans une très forte proportion des cas, et le nombre d'usages ou des détails d'usage (qui, quand, où) doivent être recueillis.

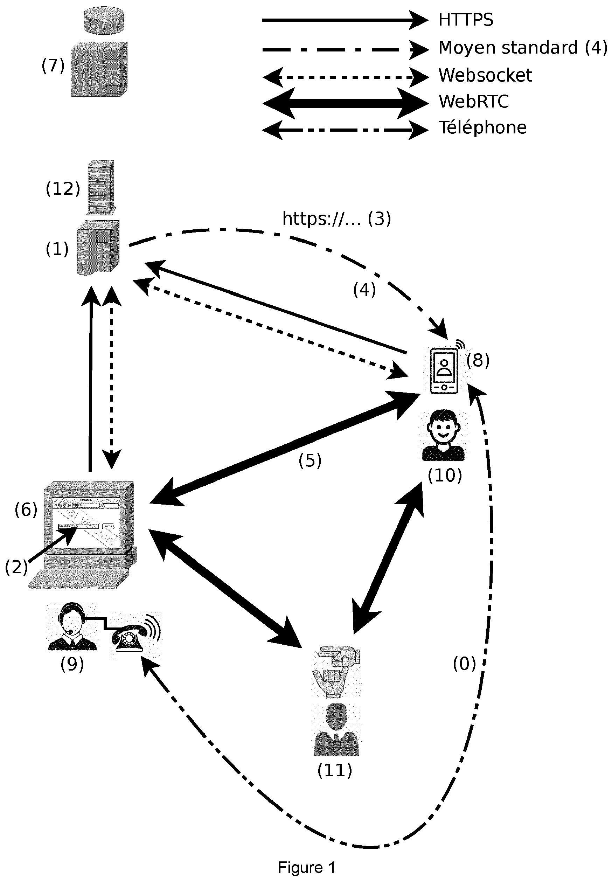

## Revendications

1. Procédé de communication de visiophonie faisant communiquer un invitant (9) avec un invité (10),
l'invité disposant d'un appareil portable (8) muni d'une caméra, d'un microphone, d'un navigateur internet, et capable de communication numérique,
l'invitant disposant d'un terminal (6) équipé d'un navigateur internet (par exemple ordinateur fixe, ordinateur portable, tablette ou éventuellement téléphone portable), d'une connexion internet, permettant de se connecter à une infrastructure (1) composée à minima d'un serveur web assurant l'accessibilité d'un site internet,
la méthode comportant les pas suivants, dans l'ordre chronologique :
- l'invitant se connecte au site internet,
- l'invitant fournit à l'infrastructure un identifiant (2) de l'invité,
- l'infrastructure génère un premier lien (3), tel qu'une URL, et transmet ce lien par un moyen numérique standard (4) à l'appareil de l'invité,
- l'invité clique sur ce lien (3),
- le navigateur de l'invité accède à la caméra et/ou au microphone de l'appareil portable (8),
- le navigateur de l'invité établit un flux visiophonique (5) avec le navigateur de l'invitant,
le procédé étant caractéristique en ce que
• le procédé ne requiert pas d'installation de logiciel spécifique, ni sur l'appareil portable (8), ni sur le terminal (6), et en ce que
• le procédé ne requiert pas d'identification de la part de l'invité (10)
• le moyen de communication standard (4) est un SMS ou un MMS ou un Webpush

2. Procédé selon la revendication 1, dans lequel le moyen de communication standard est prioritairement le Webpush si tel moyen est disponible, avec recours au SMS uniquement si le Webpush n'est pas disponible, si la demande Webpush échoue, ou s'il n'y a pas dans un délai prédéfini soit acquittement du webpush soit déclenchement de l'action souhaitée ; lequel délai peut être paramétrable.

3. Procédé selon l'une des revendications précédentes, dans lequel le premier pas de la revendication 1 est précédé par l'établissement d'une communication téléphonique (0) entre l'invité et l'invitant, celle-ci étant optionnellement maintenue pendant tout le procédé.

4. Procédé selon l'une des revendications précédentes, dans lequel l'établissement de la communication visiophonique
ne requiert de la part de l'invitant pas d'identification et/ou
ne requiert que les actions suivantes : la saisie de l'identifiant (2) ou le choix de l'invité dans une liste ; optionnellement, si et seulement si l'implémentation prévoit une communication bidirectionnelle, une autorisation d'accès aux capteurs audio et/ou vidéo de son terminal.

5. Procédé selon l'une des revendications précédentes, dans lequel l'établissement de la communication visiophonique ne requiert de la part de l'invité, outre le clic sur le lien, aucune autre action, sauf éventuellement une autorisation d'accès aux capteurs audio et/ou vidéo de son appareil portable.

6. Procédé selon l'une des revendications précédentes, dans lequel l'identifiant de l'invité est un numéro de téléphone ou une adresse email.

7. Procédé selon l'une des revendications précédentes dans lequel la communication visiophonique est mono directionnelle.

8. Procédé selon l'une des revendications précédentes dans lequel l'invitant et/ou l'invité dispose d'un moyen d'activer ou de modifier sur l'appareil portable
- le mode « mains-libre » de la conversation téléphonique et/ou
- le mode « mains-libre » de la communication visio et/ou
- le réglage du volume de la restitution sonore (haut-parleur) et/ou
- le réglage du volume de la capture sonore (niveau du micro) et/ou
- le choix d'une caméra parmi une pluralité de caméras de l'appareil portable et/ou
- le contrôle de la lumière d'éclairage « torche »
- la prise de photos ou de différents enregistrements vidéo lesquels peuvent optionnellement être numériquement signés et/ou horodatés.

9. Procédé selon l'une des revendications précédentes dans lequel des documents sont échangés pendant la communication ou ultérieurement, dans lequel optionnellement les documents sont chiffrés ou d'accès protégés par un mot de passe partagé, lequel mot de passe aura été partagé pendant la communication visio, ou sera partagé à l'issue de la communication visio, par affichage et/ou envoi de SMS et/ou Webpush, et éventuellement stocké en local par le navigateur internet en vue d'un ré-affichage ou de son usage logiciel pour déchiffrer les documents.

10. Procédé selon la revendication précédente dans lequel le mot de passe de chiffrement est annoncé par le moyen de communication standard (4), possiblement à l'avance conjointement au lien (3), ou dans lequel le mot de passe de chiffrement est l'une des constituantes du lien (3).

11. Procédé selon l'une des revendications précédentes dans lequel une génération de clés de chiffrement asymétriques a lieu dans l'un, l'autre, ou chacun des navigateurs internet et où la communication visio ou les écrans de fin de connexion est/sont le moment de partage des clés publiques, l'autre clé restant secrètement stockée localement, en vue de chiffrer/déchiffrer les échanges de documents ou de données démembrées (parties) d'un document final, ces échanges de documents ou de données démembrées étant parallèles ou ultérieurs à la communication visio.

12. Procédé selon l'une des revendications précédentes dans lequel
les documents, ou les données variables démembrées de chaque document, sont chiffrées, et font l'objet d'un transport à l'autre partie ; et dans lequel
le navigateur de l'autre partie déchiffre localement les documents chiffrés, ou les données variables démembrées qu'il ré-assemble avec une partie générique de document pour constituer localement le document final ;
la transmission des documents ou des données variables s'effectuant sous sa forme chiffrée par Webpush, websocket et/ou HTTP(s) avec éventuellement dépose sur un stockage en ligne.

13. Procédé selon l'une des revendications précédentes permettant en outre à l'une des parties de faire intervenir un interprète ou un conseiller ou un traducteur (11) ou tout autre tiers avant l'établissement ou pendant la communication visio, résultant dans l'établissement de flux en triangle chacun transmettant son flux sortant aux deux destinataires.

14. Logiciel qui, lorsqu'il est embarqué sur un serveur web, permet de réaliser le procédé selon l'une des revendications précédentes.

15. Serveur web muni d'un logiciel permettant de réaliser le procédé selon l'une des revendications précédentes.
